# EUROPÄISCHE PATENTSCHRIFT

(11) **EP 0 560 127 B1**
(45) Veröffentlichungstag und Bekanntmachung des Hinweises auf die Patenterteilung: **08.05.1996**
(21) Anmeldenummer: 93102910.2
(22) Anmeldetag: 25.02.1993
(51) Int. Cl.: C07C 209/72

(54) **Verfahren zur Herstellung von cycloaliphatischen Aminen**
Process for the preparation of cycloaliphatic amines
Procédé pour la préparation d'amines cycloaliphatiques

(30) Priorität: 07.03.1992 DE 4207314
(43) Veröffentlichungstag der Anmeldung: 15.09.1993
(73) Patentinhaber: BASF Aktiengesellschaft, D-67063 Ludwigshafen (DE)
(72) Erfinder: Wambach, Ludwig, Dr., W-6900 Heidelberg (DE); Irgang, Matthias, Dr., W-6900 Heidelberg (DE)

(56) Entgegenhaltungen:
- EP-A- 0 067 058
- EP-A- 0 324 983
- EP-A- 0 324 984
- CH-A- 445 476
- FR-A- 1 530 477
- NL-A- 6 709 040

## Beschreibung

Die vorliegende Erfindung betrifft ein druckloses Verfahren der Hydrierung aromatischer Amine zu den entsprechenden cycloaliphatischen Aminen in Gegenwart eines alkali- und/oder erdalkali-dotierten Ruthenium und Palladium oder Platin enthaltenden Katalysators.

Es ist bekannt, cycloaliphatische Amine, wie Cyclohexylamin, durch katalytische Hydrierung der entsprechenden aromatischen Amine, wie Anilin, herzustellen. Dafür sind insbesondere Rutheniumkatalysatoren (Charles L. Thomas: Catalytic Processes and proven Catalysts, Academic Press, 1970, Seite 141), Palladiumkatalysatoren (EP-A-53 818) oder Ruthenium und Palladium und/oder Platin enthaltende Katalysatoren, (Nippon Kagaku Kaishi, 1972, (5) 830 bis 836, Chem. Abstr. Vol.: 77; 47 691 f, EP-A-324 983, DE-A-38 01 755; SU-A-520 346, Chem. Abstr. Vol.: 85; 176 953 g) geeignet.

Nachteil all dieser Verfahren ist jedoch, daß nur unter recht hohen Drücken eine hinreichend lange Standzeit mit akzeptabler Aktivität und geringer Neigung zur Bildung von sekundären Aminen oder Kohlenwasserstoffen erreicht wird (P.N. Rylander: Hydrogenation Methods, Academic Press. 1985, Seite 123 bis 132).

Ein großes Problem der bisherigen Verfahrensentwicklungen bestand darin, daß die verwendeten Ru-Katalysatoren in Gegenwart von Ammoniak schnell desaktivierten oder die Reaktion stark verlangsamten (z.B. EP-A-67 058).

In der EP-A-324 984 ist ein Verfahren zur Herstellung von Dicyclohexylamin neben geringen Anteilen an Cyclohexylamin an einem Ruthenium und Palladium enthaltenden Katalysator beschrieben. Die während der Reaktion gebildeten geringen Mengen an Ammoniak werden hierbei aus dem im Kreis gefahrenen überschüssigen Wasserstoff auskondensiert.

Der vorliegenden Erfindung lag daher die Aufgabe zugrunde, den zuvor genannten Nachteilen abzuhelfen.

Demgemäß wurde ein neues und verbessertes Verfahren zur Herstellung von cycloaliphatischen Aminen der allgemeinen Formel I 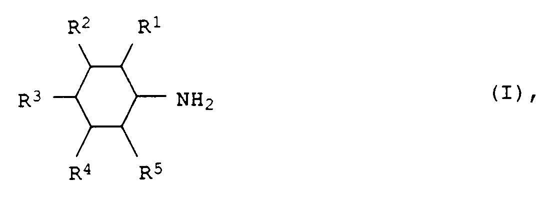
in der
- R¹,R²,R³,R⁴,R⁵: Wasserstoff, C₁- bis C₂₀-Alkyl, C₃- bis C₂₀-Cycloalkyl, C₄- bis C₂₀-Cycloalkyl-alkyl, Aryl, C₁- bis C₂₀-Aralkyl, C₇- bis C₂₀-Alkylaryl oder R¹, R² oder R², R³ gemeinsam für eine gegebenenfalls durch Sauerstoff und/oder Stickstoff unterbrochene und gegebenenfalls durch C₁- bis C₄-Alkyl substituiertes C₃- bis C₆-Alkylen bedeuten,
durch katalytische Hydrierung von aromatischen Aminen der allgemeinen Formel II 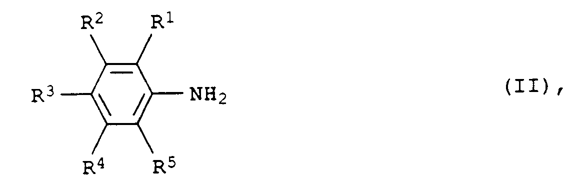
in der R¹, R², R³, R⁴ und R⁵ die oben genannten Bedeutungen haben, gefunden, welches dadurch gekennzeichnet ist, daß man die Umsetzung mit Wasserstoff in Gegenwart von Ammoniak an alkali- und/oder erdalkali-dotierten Katalysatoren, die Ruthenium und Palladium oder Platin auf Trägern enthalten bei Temperaturen von 120 bis 250°C und Drücken von 0,1 bis 5 bar.

Das erfindungsgemäße Verfahren läßt sich z. B. wie folgt durchführen:
Zuerst wird Anilin und gegebenenfalls zurückgeführtes Dicyclohexylamin mit Kreisgas vermischt und in einem Vorheizer vollständig verdampft. Die Temperatur des Gasgemisches wird so eingestellt, daß die erforderliche Reaktionstemperatur im Reaktor erreicht wird. Anschließend leitet man das Gasgemisch über ein Katalysatorbett. Dort wird die Temperatur durch Kühlung auf Reaktionstemperatur gehalten. Schließlich kondensiert man das Produkt aus, führt es einer Destillation zu und leitet das Kreisgas zurück.

Der erfindungsgemäße Katalysator enthält die Edelmetalle Ruthenium zu Palladium oder Platin in einer Gesamtmenge von 0,2 bis 5 Gew.%, bevorzugt 0,5 bis 2 Gew.% und in einem Gewichtsverhältnis von 0,2 : 1 bis 5 : 1, bevorzugt 0,5 : 1 bis 4 : 1, sowie eine Alkali- und/oder Erdalkaliverbindung bezogen auf das Gesamtgewicht der Katalysators von 0,1 bis 10 Gew.%, bevorzugt 0,5 bis 3 Gew.% und einem Träger besteht.

Als Alkaliverbindung kommen in Frage die basischen Oxide, Carbonate oder Hydroxyde der Alkalimetalle, wobei insbesondere die von Lithium, Natrium und Kalium bevorzugt sind.

Als Erdalkaliverbindung kommen in Frage die Chloride oder basischen Oxide, Carbonate oder Hydroxyde der Erdalkalimetalle, wobei insbesondere die des Magnesiums, Bariums und Calciums bevorzugt sind.

Als Trägermaterialien kommen in Frage Aluminiumoxide, z.B. D 10 - 10 (R) oder D 10 - 20 (R) , Aktivkohle, Bariumsulfat, Titandioxid, Kieselgur, Silicagel, Calciumcarbonat, Bariumcarbonat, aber auch andere handelsübliche Trägermaterialien mit entsprechender Härte und Temperaturbeständigkeit wie z.B. Zeolithe.

Die oben beschriebenen Katalysatoren des erfindungsgemäßen Verfahrens können hergestellt werden nach allgemein bekannten Verfahren, wie z.B. im Gmelin, Erg. 63, Seite 173 bis 176 und der dort zitierten Literaturstellen sowie US-A-3 183 278 aufgezeigt. Dabei ist es möglich, die Alkali- oder Erdalkaliverbindung nach allgemein bekannten Verfahren gleichzeitig mit den Edelmetallen oder in einem gesonderten Arbeitsgang aufzutränken. Werden die Edelmetalle in Form ihrer Hydroxide aufgetragen, so ist es zweckmäßig, sie in Suspension aufzusprühen. In diesem Fall kann auf die zusätzliche Auftränkung einer Alkali-und/oder Erdalkaliverbindung verzichtet werden. Der Katalysator wird bevorzugt im Reaktor vor seinem Einsatz durch Behandeln mit Wasserstoff während mehrerer Stunden bei erhöhter Temperatur aktiviert. Eine solch erhöhte Temperatur liegt beispielsweise zwischen 140 und 340°C.

Die Substituenten R¹, R², R³, R⁴ und R⁵ der Verbindungen I und II haben folgende Bedeutungen:
R¹,R²,R³,R⁴,R⁵
- unabhängig voneiander
- Wasserstoff,
- C₁- bis C₂₀-Alkyl, bevorzugt C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₁- bis C₂₀-Cycloalkyl, bevorzugt C₃- bis C₈-Cycloalkyl wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
- C₄- bis C₂₀-Cycloalkyl-alkyl,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇- bis C₂₀-Alkylaryl, bevorzugt C₇- bis C₁₂-Alkylphenyl wie 2-Methylphenyl, 3-Methylphenyl, 4-Methylphenyl, 2,4-Dimethyl-phenyl, 2,5-Dimethylphenyl, 2,6-Dimethylphenyl, 3,4-Dimethyl-phenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
- C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
R¹,R² oder R²,R³
- gemeinsam
- ein gegebenenfalls durch C₁- bis C₄-Alkyl substituiertes C₃- bis C₆-Alkylen, wie -(CH₂)₃-, -(CH₂)₄-, -(CH₂)₅-, -(CH₂)₆-, -CH(CH₃)-CH₂-CH₂- und -CH₂-CH(CH₃)-CH₂-,
- ein durch Sauerstoff und/oder Stickstoff unterbrochene und gegebenenfalls durch C₁- bis C₄-Alkyl substituiertes C₃- bis C₆-Alkylen.

Die oben beschriebenen Katalysatoren können in hervorragender Weise zur Hydrierung von aromatischen Aminen, insbesondere von Anilin, zu den entsprechenden cycloaliphatischen Aminen, insbesondere Cyclohexylamin, in Gegenwart von Ammoniak, bei tiefen Temperaturen und Normaldruck verwendet werden, wobei die Bildung von sekundären Aminen weitgehendst unterdrückt wird. Die erfindungsgemäßen Katalysatoren zeichnen sich durch eine bei Normaldruck in der Gasphase in Gegenwart größerer Mengen an Ammoniak durchgeführten Hydrierung von Anilin und anderen aromatischen Aminen noch nicht beobachteten Standzeit bei gleichzeitig hervorragender Selektivität zu Cyclohexylamin bzw. den entsprechenden cycloaliphatischen Aminen aus. Die Bildung von Nebenprodukten wie Kohlenwasserstoffen oder N-Phenylcyclohexyl-aminen ist gering.

Um eine gute Selektivität der erfindungsgemäßen Katalysatoren zu cycloaliphatischen Aminen, insbesondere Cyclohexylamin, zu erhalten, sollte das Wasserstoff/Ammoniak-Verhältnis kleiner 5 zu 1, bevorzugt kleiner 2,5 zu 1, sein. Das bei der Reaktion nicht verbrauchte Gas kann im Kreis zurückgeführt werden. Die Temperatur des erfindungsgemäßen Verfahrens beträgt in der Regel zwischen 120 und 250°C, bevorzugt zwischen 150 und 210°C und die Drücke zwischen 0,1 bis 5 bar, bevorzugt 0,5 bis 2 bar, besonders bevorzugt bei Atmosphärendruck (Normaldruck). Eine höhere Reaktortemperatur bedingt eine Erhöhung des Cyclohexylamin- und Kohlenwasserstoffanteils im Reaktionsaustrag.

Die Katalysatorbelastung soll 0,2 kg Anilin pro Stunde und Liter Katalysator, vorzugsweise 0,1 kg, nicht übersteigen. Zur Erreichung dieser langen Standzeit ist es wichtig, die anfängliche Katalysatorbelastung nicht zu hoch zu wählen.

Kommt es im Laufe der Zeit dennoch zu einer Desaktivierung des Katalysators, so ist es zweckmäßig, den auf dem Katalysator abgeschiedenen Kohlenstoffbelag durch Behandeln mit einer Stickstoff/Luft-Mischung bei erhöhter Temperatur zu verbrennen. Eine solch erhöhte Temperatur kann z.B. zwischen 250 und 450°C liegen. Durch anschließende Reduktion des Katalysators kann dieser wieder seine volle Aktivität erreichen.

Vorteil des erfindinngsgemäßen Katalysators ist die drucklose Reaktionsführung und dennoch sehr lange Standzeit in Gegenwart größerer Mengen an Ammoniak, sowie die bei dieser Reaktionsführung geringe Neigung zur Bildung sekundärer Amine bei sehr gutem Umsatz und kurzen Verweilzeiten.

Cyclohexylamin und substituierte Cyclohexylamine finden z.B. Verwendung auf dem Textilhilfsmittelsektor, Pflanzenschutzmittel- sektor, als Korrosionsschutzmittel und als Monomere für die Kunststoffe (Ullmanns Enzyklopädie der techn. Chemie, 3. Auflage, Band 5,Seiten 681 bis 685).

### Katalysatorherstellung

### Katalysator A

Kugelförmiger Katalysatorträger, bestehend aus Gamma-Al₂O₃, wird mit einer wässrigen NaOH-Lösung so getränkt, daß alle Poren gefüllt sind. Die feuchten Kugeln werden im Trockenschrank bei ca. 120°C getrocknet und enthalten dann 3.10 % Na₂O bezogen auf das wasserfreie Produkt. Anschließend wird eine Mischlösung von Palladiumchlorid und Rutheniumchlorid hergestellt und die zugesetzte Wassermenge so berechnet, daß durch das Lösungsvolumen wiederum Porenfüllung erreicht wird. Der oben beschriebene Na₂O-haltige Träger wird in einer Imprägniertrommel vorgelegt und die Ru-Pd-Lösung innerhalb von 60 Minuten aufgesprüht. Das erhaltene Produkt wird innerhalb von 16 Stunden bei 120°C getrocknet und enthält 1,4 % Ru, 1,1 % Pd und 2,9 % Na₂O.

### Katalysator B

Zunächst wird ein Na₂O-haltiger Träger aus Gamma-Al₂O₃-Kugeln und NaOH hergestellt, wie bei Katalysator A beschrieben. Man stellt eine Mischlösung aus Rutheniumchlorid und Palladium-chlorid her und verdünnt diese auf das zur Porenfüllung erfor-derliche Volumen. Mit dieser Lösung wird der in einer Imprägniertrommel befindliche Träger besprüht. Nach der Trocknung enthält der Katalysator 2,9 % Na₂O, 0,5 % Ru und 0,5 % Pd.

### Katalysator C

Ein kugelförmiger Katalysatorträger, bestehend aus Gamma-Al₂O₃ und 3,1 Gew.% Na₂O wird - wie bei Katalysator A beschrieben - hergestellt. Man stellt eine Mischlösung aus Rutheniumchlorid, Palladiumchlorid und Magnesiumchlorid her. Diese Lösung wird auf den in einer Imprägniertrommel befindlichen Träger innerhalb von 60 Minuten aufgesprüht. Das Produkt wird bei 120°C im Trocken-schrank getrocknet und enthält 0,7 % Ru; 0,7 % Pd; 0,3 % Mg und 3,0 % Na₂O.

### Katalysator D

Strangförmiger Katalystorträger, bestehend aus fast gleichen Teilen Gamma-Al₂O₃ und Ba0 wird in einer Imprägniertrommel vorgelegt. Dann wird eine Suspension aus Palladiumchlorid, Rutheniumhydroxid-Paste und VE-Wasser bei 120 - 150°C auf den Träger aufgesprüht. Die Suspension darf sich während des Sprühens nicht absetzen. Das erhaltene Produkt wird innerhalb von 16 Stunden bei 120°C getrocknet und enthält 0,7 % Pd und 0,3 % Ru.

### Katalysator E

Kugelförmiger Katalysatorträger, bestehend aus Gamma-Al₂O₃, wird mit einer wässrigen NaOH-Lösung so getränkt, daß alle Poren gefüllt sind. Die feuchten Kugeln werden im Trockenschrank bei 120°C getrocknet und enthalten dann 3,1 % Na₂O bezogen auf das wasserfreie Produkt. Anschließend wird eine Mischlösung von Platinchlorid, Rutheniumchlorid und Magnesiumchlorid hergestellt und die zugesetzte Wassermenge so berechnet, daß durch das Lösungsvolumen wiederum Porenfüllung erreicht wird. Der oben beschriebene Na₂O-haltige Träger wird in einer Imprägniertrommel vorgelegt und die Ru-Pt-Mg-Lösung innerhalb von 60 Minuten aufgesprüht. Das Produkt wird bei 120°C im Trockenschrank getrocknet und enthält 0,7 % Ru; 0,2 % Pt; 0,3 % Mg und 3,0 % Na₂O.

### Katalysator F

Man stellt eine Mischlösung aus Rutheniumchlorid, Palladiumchlorid und Magnesiumchlorid her. Diese Lösung wird auf den in einer Imprägniertrommel befindlichen strangförmigen Träger, bestehend aus Bariumcarbonat, innerhalb von 60 Minuten auf-gesprüht. Das Produkt wird bei 120°C im Trockenschrank getrocknet und enthält 0,7 % Ru; 0,7 % Pd und 0,3 % Mg.

### Beispiele

### Beispiel 1

300 ml des oben beschriebenen Katalysators A werden in ein senkrecht angeordnetes Reaktionsrohr mit einem Durchmesser von 50 mm eingefüllt und mit 100 l/h Wasserstoff aktiviert. Dazu wird die Temperatur innerhalb von 6 Stunden auf 150°C angehoben und dort 27 Stunden gehalten. Dann werden pro Stunde 20 ml Anilin, 40 l Ammoniak und 60 l Wasserstoff bei 160°C über den Katalysator gefahren. Aus dem Reaktionsaustrag werden die bei Raumtemperatur flüssigen Bestandteile auskondensiert und das Gas mit 300 l/h als Kreisgas rückgeführt. Das kondensierte Reaktionsprodukt wird in verschiedenen Zeitabständen analysiert. Dabei werden in Abhängigkeit von den Betriebsstunden des Katalysators und der Reaktionstemperatur folgende Zusammensetzungen des Reaktionsproduktes gefunden:

| Versuchsdauer | 24 h | 240 h | 600 h | 850 h | 1 200 |
|---|---|---|---|---|---|
| Cyclohexan | 0,32 % | 0,2 % | 0,2 % | 0,4 % | 0,2 % |
| Benzol | 0,5 % | 0,6 % | 0,5 % | 0,8 % | 0,5 % |
| Cyclohexylamin | 78,8 % | 76,5 % | 80,4 % | 82,5 % | 80,9 % |
| Dicyclohexylamin | 16 % | 16,5 % | 14,8 % | 11,5 % | 14,5 % |
| Anilin | 4,1 % | 5,9 % | 4 % | 4,5 % | 3,8 % |
| N-Phenylcyclohexylamin | 0,1 % | 0,1 % | 0,1 % | 0,2 % | 0 % |
| Reaktionstemperatur | 160°C | 160°C | 160°C | 170°C | 160°C |

### Beispiel 2

Mit dem oben beschriebenen Katalysator B wird verfahren wie in Beispiel 1. Allerdings vollzog sich die Aktivierung des Katalysators innerhalb von 16 Stunden bei 250°C. In Abhängigkeit von den Betriebsstunden des Katalysators und der Reaktionstemperatur werden folgende Zusammensetzungen des Reaktionsproduktes gefunden:

| Versuchsdauer | 24 h | 300 h | 400 h | 500 h |
|---|---|---|---|---|
| Cyclohexan | 0,1 % | 0,1 % | 0,1 % | 0,1 % |
| Benzol | 0,17 % | 0,24 % | 0,17 % | 0,23 % |
| Cyclohexylamin | 73,6 % | 78,5 % | 75,5 % | 76,1 % |
| Dicyclohexylamin | 22,9 % | 15,5 % | 20,0 % | 18,0 % |
| Anilin | 3,2 % | 5,5 % | 4,2 % | 5,5 % |
| N-Phenylcyclohexylamin | 0,01 % | 0,1 % | 0,1 % | 0,1 % |
| Reaktionstemperatur | 160°C | 170°C | 170°C | 170°C |

### Beispiel 3

Mit dem oben beschriebenen Katalysator C wird verfahren wie in Beispiel 2. In Abhängigkeit von den Betriebsstunden des Katalysators und der Reaktionstemperatur werden folgende Zusammensetzungen des Reaktionsaustrags gefunden:

| Versuchsdauer | 200 h |
|---|---|
| Anilinzulauf | 25 g/h l |
| Cyclohexan | 0,3 % |
| Benzol | 0,4 % |
| Cyclohexylamin | 84,0 % |
| Dicyclohexylamin | 14,8 % |
| Anilin | 0,5 % |
| N-Phenylcyclohexylamin | 0,02 % |
| Reaktionstemperatur | 160°C |

### Beispiel 4

Mit dem oben beschriebenen Katalysator D wird verfahren wie im Beispiel 2.

In Abhängigkeit von den Betriebsstunden des Katalysators und der Reaktionstemperatur werden folgende Zusammensetzungen des Reaktionsaustrags gefunden:

| Versuchsdauer | 24 h | 250 h | 500 h |
|---|---|---|---|
| Anilinzulauf | 26 g/l h | 36 g/l h | 36 g/l h |
| Cyclohexan | --- | 0,1 | 0,1 |
| Benzol | 0,3 | 0,4 | 0,8 |
| Cyclohexylamin | 59 | 66,8 | 65,1 |
| Dicyclohexylamin | 19 | 17,5 | 16,7 |
| Anilin | 21 | 14,5 | 16 |
| N-Phenylcyclohexylamin | 0,7 | 0,7 | 1,2 |
| Reaktionstemperatur | 190°C | 190°C | 190°C |

### Beispiel 5

Mit dem oben beschriebenen Katalysator E wird verfahren wie inBeispiel 2. In Abhängigkeit von den Betriebsstunden des Katalysators, des Zulaufs an Anilin und der Reaktionstemperatur werden folgende Zusammensetzungen des Reaktionsaustrags gefunden:

| Versuchsdauer | 24 h | 210 h | 1000 h | 2000 h |
|---|---|---|---|---|
| Anilinzulauf | 35 g/l h | 50 g/l h | 50 g/l h | 50 g/l h |
| Cyclohexon | 0,5 | 0,4 | 0,3 | 0,2 |
| Benzol | 0,4 | 0,6 | 0,6 | 0,6 |
| Cyclohexylamin | 80,3 | 84,1 | 82,6 | 82,8 |
| Dicyclohexylamin | 18,9 | 14,8 | 15,8 | 15,6 |
| Anilin | 0,01 | 0,1 | 0,7 | 1,1 |
| N-Phenylcyclohexylamin | --- | --- | --- | --- |
| Reaktionstemperatur | 160°C | 160°C | 160°C | 160°C |

### Beispiel 6

Mit dem oben beschriebenen Katalysator F wird verfahren wie in Beispiel 2.

In Abhängigkeit von den Betriebsstunden des Katalysators, des Zulaufs an Anilin und der Reaktionstemperatur werden folgende Zusammensetzungen des Reaktionsaustrags gefunden:

| Versuchsdauer | 24 h | 100 h | 250 h | 500 h |
|---|---|---|---|---|
| Anilinzulauf | 35 g/l h | 50 g/l h | 50 g/l h | 50 g/l h |
| Cyclohexan | 0,2 | 0,1 | 0,15 | 0,1 |
| Benzol | 0,15 | 0,2 | 0,2 | 0,3 |
| Cyclohexylamin | 88,6 | 85,1 | 85,1 | 84,7 |
| Dicyclohexylamin | 11 | 12,9 | 12,3 | 12,2 |
| Anilin | 0,05 | 1,6 | 2,2 | 2,6 |
| N-Phenylcyclohexylamin | --- | --- | --- | --- |
| Reaktionstemperatur | 160°C | 160°C | 170°C | 170°C |

## Patentansprüche

1. Verfahren zur Herstellung von cycloaliphatischen Aminen der allgemeinen Formel I 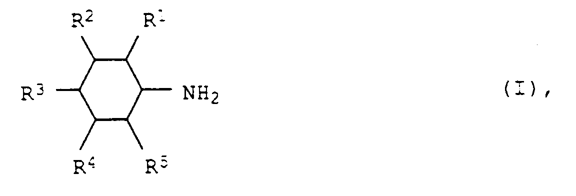 in der
R¹,R²,R³,R⁴,R⁵ Wasserstoff, C₁- bis C₂₀-Alkyl, C₃- bis C₂₀-Cycloalkyl, C₄- bis C₂₀-Cycloalkyl-alkyl, Aryl, C₁- bis C₂₀-Aralkyl, C₇- bis C₂₀-Alkylaryl oder R¹, R² oder R², R³ gemeinsam für eine gegebenenfalls durch Sauerstoff und/oder Stickstoff unterbrochene und gegebenenfalls durch C₁- bis C₄-Alkyl substituiertes C₃- bis C₆-Alkylen
bedeuten, durch katalytische Hydrierung von aromatischen Aminen der allgemeinen Formel II 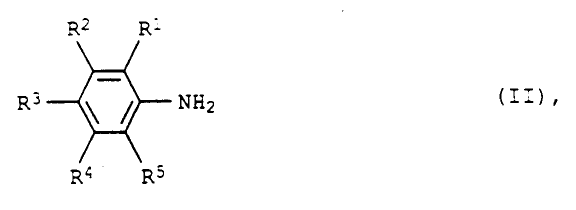 in der R¹, R², R³, R⁴ und R⁵ die oben genannten Bedeutungen haben, dadurch gekennzeichnet, daß man die Umsetzung mit Wasserstoff in Gegenwart von Ammoniak an alkali- und/oder erdalkali-dotierten Katalysatoren, die Ruthenium und Palladium oder Platin auf Trägern enthalten bei Temperaturen von 120 bis 250 °C und Drücken von 0,1 bis 5 bar durchführt.

2. Verfahren zur Herstellung von cycloaliphatischen Aminen der allgemeinen Formel I nach Anspruch 1, dadurch gekennzeichnet, daß der alkali- und/oder erdalkali-dotierte Katalysator Ruthenium und Palladium oder Platin in einer Gesamtmenge von 0,2 bis 5 Gew.% enthält.

3. Verfahren zur Herstellung von cycloaliphatischen Aminen der allgemeinen Formel I nach Anspruch 1, dadurch gekennzeichnet, daß der alkali- und/oder erdalkali-dotierte Katalysator Ruthenium und Palladium oder Platin in einer Gesamtmenge von 0,5 bis 2 Gew.% enthält.

4. Verfahren zur Herstellung von cycloaliphatischen Aminen der allgemeinen Formel I nach Anspruch 1, dadurch gekennzeichnet, daß der alkali- und/oder erdalkali-dotierte Katalysator Ruthenium und Palladium oder Platin in einem Gewichtsverhältnis 0,2 : 1 bis 5 : 1 enthält.

5. Verfahren zur Herstellung von cycloaliphatischen Aminen der allgemeinen Formel I nach Anspruch 1, dadurch gekennzeichnet, daß der alkali- und/oder erdalkali-dotierte Katalysator Ruthenium und Palladium oder Platin in einem Gewichtsverhältnis 0,5 : 1 bis 4 : 1 enthält.

6. Verfahren zur Herstellung von cycloaliphatischen Aminen der allgemeinen Formel I nach Anspruch 1, dadurch gekennzeichnet, daß der alkali- und/oder erdalkali-dotierte Katalysator Ruthenium und Palladium oder Platin 0,1 bis 10 Gew.% einer Alkali- und/oder Erdalkali-metallverbindung enthält.

7. Verfahren zur Herstellung von cycloaliphatischen Aminen der allgemeinen Formel I nach Anspruch 1, dadurch gekennzeichnet, daß der alkali- und/oder erdalkali-dotierte Katalysator Ruthenium und Palladium oder Platin 0,5 bis 3 Gew.% einer Alkali- und/oder Erdalkali-metallverbindung enthält.

8. Verfahren zur Herstellung von cycloaliphatischen Aminen der allgemeinen Formel I nach Anspruch 1, dadurch gekennzeichnet, daß das Wasserstoff/Ammoniak-Verhältnis zwischen 5 : 1 und 1 : 1 beträgt.

9. Verfahren zur Herstellung von cycloaliphatischen Aminen der allgemeinen Formel I nach Anspruch 1, dadurch gekennzeichnet, daß das Wasserstoff/Ammoniak-Verhältnis zwischen 2,5 : 1 und 1 : 1 beträgt.

10. Verfahren zur Herstellung von cycloaliphatischen Aminen der allgemeinen Formel I nach Anspruch 1, dadurch gekennzeichnet, daß man die Umsetzung bei Temperaturen zwischen 150 und 210°C durchführt.

## Claims

1. A process for preparing cycloaliphatic amines of the general formula I 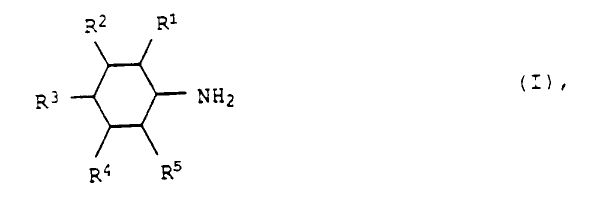 where
R¹, R², R³, R⁴, R⁵ are hydrogen, C₁-C₂₀-alkyl, C₃-C₂₀-cycloalkyl, C₄-C₂₀-cycloalkylalkyl, aryl, C₁-C₂₀-aralkyl, C₇-C₂₀-alkylaryl or R¹, R² or R², R³ are together C₃-C₆-alkylene which may be uninterrupted or interrupted by oxygen and/or nitrogen and may be unsubstituted or substituted by C₁-C₄-alkyl,
by catalytic hydrogenation of aromatic amines of the general formula II 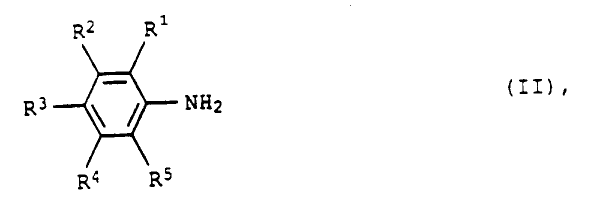 where R¹, R², R³, R⁴ and R⁵ are as defined above, wherein the reaction with hydrogen is carried out in the presence of ammonia over alkali metal-doped and/or alkaline earth metal-doped catalysts comprising ruthenium and palladium or platinum on supports, at from 120 to 250°C and pressures of from 0.1 to 5 bar.

2. A process for preparing cycloaliphatic amines of the general formula I as claimed in claim 1, wherein the alkali metal-doped and/or alkaline earth metal-doped catalyst contains ruthenium and palladium or platinum in a total amount of from 0.2 to 5% by weight.

3. A process for preparing cycloaliphatic amines of the general formula I as claimed in claim 1, wherein the alkali metal-doped and/or alkaline earth metal-doped catalyst contains ruthenium and palladium or platinum in a total amount of from 0.5 to 2% by weight.

4. A process for preparing cycloaliphatic amines of the general formula I as claimed in claim 1, wherein the alkali metal-doped and/or alkaline earth metal-doped catalyst contains ruthenium and palladium or platinum in a weight ratio of from 0.2:1 to 5:1.

5. A process for preparing cycloaliphatic amines of the general formula I as claimed in claim 1, wherein the alkali metal-doped and/or alkaline earth metal-doped catalyst contains ruthenium and palladium or platinum in a weight ratio of from 0.5:1 to 4:1.

6. A process for preparing cycloaliphatic amines of the general formula I as claimed in claim 1, wherein the alkali metal-doped and/or alkaline earth metal-doped catalyst comprises ruthenium and palladium or platinum and from 0.1 to 10% by weight of an alkali metal and/or alkaline earth metal compound.

7. A process for preparing cycloaliphatic amines of the general formula I as claimed in claim 1, wherein the alkali metal-doped and/or alkaline earth metal-doped catalyst comprises ruthenium and palladium or platinum and from 0.5 to 3% by weight of an alkali metal and/or alkaline earth metal compound.

8. A process for preparing cycloaliphatic amines of the general formula I as claimed in claim 1, wherein the hydrogen/ammonia ratio is from 5:1 to 1:1.

9. A process for preparing cycloaliphatic amines of the general formula I as claimed in claim 1, wherein the hydrogen/ammonia ratio is from 2.5:1 to 1:1.

10. A process for preparing cycloaliphatic amines of the general formula I as claimed in claim 1, wherein the reaction is carried out at from 150 to 210°C.

## Revendications

1. Procédé de préparation d'amines cycloaliphatiques de formule générale I 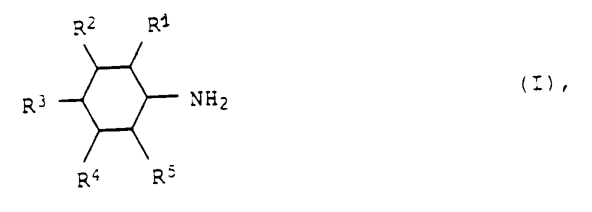 dans laquelle
R¹,R²,R³,R⁴,R⁵ représentent des atomes d'hydrogène, des restes alkyle en C₁-C₂₀, cycloalkyle en C₃-C₂₀, aryle, aralkyle en C₁-C₂₀, alkylaryle en C₇-C₂₀, ou bien R¹, R² ou R², R³ sont mis ensemble pour un reste alkylène en C₃-C₆ éventuellement interrompu par un atome d'oxygène et/ou d'azote et éventuellement substitué par un reste alkyle en C₁-C₄,
par hydrogénation catalytique d'amines aromatiques de formule générale II 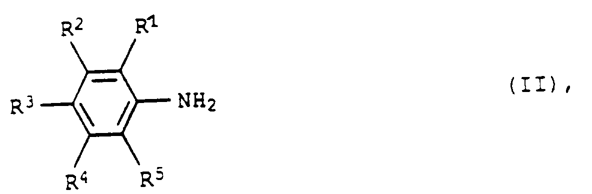 dans laquelle R¹, R², R³, R⁴ et R⁵ ont les significations données ci-dessus, caractérisé en ce que l'on conduit la réaction avec de l'hydrogène en présence d'ammoniac et de catalyseurs dopés avec des métaux alcalins et/ou alcalino-terreux qui contiennent du ruthénium et du palladium ou du platine sur des supports, à des températures de 120 à 250°C et sous des pressions de 0,1 à 5 bar.

2. Procédé de préparation d'amines cycloaliphatiques de formule générale I selon la revendication 1, caractérisé en ce que le catalyseur dopé avec des métaux alcalins et/ou alcalino-terreux contient du ruthénium et du palladium ou du platine dans une proportion totale de 0,2 à 5% en poids.

3. Procédé de préparation d'amines cycloaliphatiques de formule générale I selon la revendication 1, caractérisé en ce que le catalyseur dopé avec des métaux alcalins et/ou alcalino-terreux contient du ruthénium et du palladium ou du platine dans une proportion totale de 0,5 à 2% en poids.

4. Procédé de préparation d'amines cycloaliphatiques de formule générale I selon la revendication 1, caractérisé en ce que le catalyseur dopé avec des métaux alcalins et/ou alcalino-terreux contient du ruthénium et du palladium ou du platine dans un rapport pondéral de 0,2:1 à 5:1.

5. Procédé de préparation d'amines cycloaliphatiques de formule générale I selon la revendication 1, caractérisé en ce que le catalyseur dopé avec des métaux alcalins et/ou alcalino-terreux contient du ruthénium et du palladium ou du platine dans un rapport pondéral de 0,5:1 à 4:1.

6. Procédé de préparation d'amines cycloaliphatiques de formule générale I selon la revendication 1, caractérisé en ce que le catalyseur dopé avec des métaux alcalins et/ou alcalino-terreux contient du ruthénium et du palladium ou du platine avec 0,1 à 10% en poids d'un composé de métal alcalin et/ou alcalino-terreux.

7. Procédé de préparation d'amines cycloaliphatiques de formule générale I selon la revendication 1, caractérisé en ce que le catalyseur dopé avec des métaux alcalins et/ou alcalino-terreux contient du ruthénium et du palladium ou du platine avec 0,5 à 3% en poids d'un composé de métal alcalin et/ou alcalino-terreux.

8. Procédé de préparation d'amines cycloaliphatiques de formule générale I selon la revendication 1, caractérisé en ce que le rapport hydrogène/ammoniac est compris entre 5:1 et 1:1.

9. Procédé de préparation d'amines cycloaliphatiques de formule générale I selon la revendication 1, caractérisé en ce que le rapport hydrogène/ammoniac est compris entre 2,5:1 et 1:1.

10. Procédé de préparation d'amines cycloaliphatiques de formule générale I selon la revendication 1, caractérisé en ce que l'on conduit la réaction à une température comprise entre 150 et 210°C.
